# EUROPEAN PATENT APPLICATION

(11) **EP 2 942 052 A1**
(43) Date of publication of application: **11.11.2015**
(21) Application number: 13870236.0
(22) Date of filing: 07.01.2013
(51) Int. Cl.: A61K 9/16, A61K 9/14, A61K 47/36, A61K 47/30

(54) **NOVEL FAST-DISSOLVING GRANULE FORMULATION HAVING IMPROVED SOLUBILITY**

(71) Applicant: Sam-A Pharm. Co. Ltd., Gangwon-do 220-801 (KR)
(72) Inventor: LEE, Jong Hoon, Suwon-si Gyeonggi-do 441-400 (KR); SHIM, Sang Bo, Anyang-si Gyeonggi-do 430-015 (KR); KIM, Sang Wook, Suwon-si Gyeonggi-do 443-400 (KR); YI, Hong Gi, Suwon-si Gyeonggi-do 440-303 (KR)
(74) Representative: Louis Pöhlau Lohrentz
(86) International application number: PCT/KR2013/000107
(87) International publication number: WO 2014/106962

(57) **Abstract**

The present invention relates to a fast-dissolving granule formulation which is dissolved quickly in the mouth. The present invention provides a fast-dissolving granule formulation which quickly dissolves within 20 seconds in the mouth when administered orally, and which causes no feelings of irritation resulting from foreign matter or residue. The granule formulation of the present invention is obtained by assembling a fast-dissolving carrier and an active ingredient, wherein the fast-dissolving carrier satisfies the conditions of having an instantaneous solubility of 30mg/ml or more, solubility of 50mg/ml or more within five minutes, and the instantaneous solubility is 90% or less than the maximum solubility. The granule formulation may achieve effects of the present invention regardless of the type of the active ingredient and therefore can be applied to various active ingredients. Further, since the present invention provides a relatively simple preparation process, a high efficiency process can be constructed at a low cost.

## Description

### [Technical Field]

The present invention relates to a novel rapidly dissolving granule formulation characterized by rapid dissolution in the oral cavity. Specifically, the present invention relates to a novel rapidly dissolving granule formulation characterized by rapid dissolution in the oral cavity within 20 seconds after oral administration without leaving any feeling of foreign matter and a residual feeling.

More specifically, the present invention relates to a novel rapidly dissolving granule formulation that contains the active ingredient together with a rapidly dissolving carrier meeting particular requirements wherein the dissolution and absorption of the active ingredient are promoted, and the granule formulation gives a feeling of refreshment without leaving a feeling of foreign matter and a residual feeling in the oral cavity.

### [Background Art]

The most popular formulations for oral administration are tablets and capsules. However, an rapidly dissolving and/or disintegrating preparations in oral cavity can be considered in case of patients having a difficulty in swallowing or if administration without taking water is needed. Examples of these rapidly dissolving and/or disintegrating preparations in oral cavity include ODT(orally disintegrating tablet) or OST(oral strip technologies). However, it generally takes at least 30 seconds for the orally rapidly disintegrating tablet to be completely disintegrated and dissolved in the oral cavity. Particularly, the orally rapidly disintegrating tablet causes a patient to feel a severe feeling of foreign matter in the oral cavity before completion of disintegration. The patient can take the orally rapidly disintegrating tablet without water, but administration convenience of the tablet is not substantially enhanced. If an active ingredient of the orally rapidly disintegrating tablet has a bitter taste, an additional technique should be applied to mask the bitter taste. Oral Strip formulations(OST) are produced by loading active ingredients in film-shaped strips. Strip formulations have been used as mouthwashes, etc., but the amount of active ingredients loaded therein is fundamentally limited. Further, sticky polymers forming the films cause inconvenience for patients due to their feeling of foreign matter and residual feeling in the oral cavities.

On the other hand, there has been a conventional granule formulations that contain herbal or plants extracts. However, since most of these conventional granule formulations are dissolved or suspended in beverage or water before administration, they are not substantially classified into formulations that can be taken without water. If a granule formulation needing mixing with beverage or water for administration is taken without water, a feeling of foreign matter and a residual feeling are severely left in the oral cavity, inevitably leading to deterioration of medication compliance.

For example, U.S. Patent Publication No. 2011/0027377 discloses a granule formulation that is produced by granulation of a sparingly soluble drug classifiable as Class II or Class IV of the Biopharmaceutical Classification System (BCS) to achieve increased solubility of the drug. This formulation is produced by mixing maltodextrin and polyethylene glycol having a particular molecular weight, together with the active ingredient, extruding the mixture using a screw extruder, and granulating the extrudate. The granules have a size of 100 to 2,500 µm. The granule formulation is dissolved in water and administered to animals. Alternatively, the granule formulation is compressed to produce a tablet or is filled in a capsule. That is, it is difficult to regard the granule formulation as an independent formulation. No disclosure is found regarding means for the enhancement of administration convenience.

U.S. patent publication No. 2011/0117193 discloses rapidly dissolving granule formulations of an antiretroviral drug for the treatment of children exposed to HIV/AIDS. The rapidly dissolving granule formulations are produced using saccharin as a sweetener and Ludiflash, croscarmellose sodium and Kollidon as disintegrants. This patent publication describes the administration of a suspension of the granules in water as a final dosage form but fails to disclose a granule formulation that can be taken without water.

Korean unexamined Patent Publication No. 10-2007-0062978 discloses granules containing paracetamol, NSAID and a sugar alcohol, made by melt extrusion. Specifically, the granules are produced by a process involving: dry blending alcohol, paracetamol and NSAID salts, optionally with other excipients which may be present in the granular component; heating the mixture at a temperature of 100 to 165 °C in an extruder to melt the sugar alcohol fully, thereby mixing the molten sugar alcohol with non-molten NSAID salts, paracetamol and other optional excipients; pouring the molten mass onto cooled stainless steel trays or a cooled moving belt at 10 °C to allow the molten mass to cool; solidifying the molten mixture; and milling the solid mass. However, this patent publication discloses the use of the granules as intermediates for the production of tablets but does not disclose any technical idea to use the granules as final formulations. Special consideration is needed regarding high production costs and equipment involved in the processes of melting the sugar alcohol at a high temperature, mixing the molten sugar alcohol with the active ingredients and cooling the mixture.

Korean unexamined Patent Publication No. 10-2009-0086469 discloses a powder formulation for valganciclovir. This formulation includes valganciclovir, povidone, fumaric acid, sodium benzoate, saccharin and mannitol. The formulation is also mixed with a particular solution before administration. Therefore, the final dosage form is not a granule formulation but a liquid formulation.

U.S. Patent No. 5,169,643 discloses a thiolcarbamate-containing granule formulation and U.S. Patent No. 5,532,209 discloses a propanil-containing granule formulation. These granule formulations do not take into account rapid dissolution and administration convenience because they are not applied to drugs for administration to humans.

As is evident from the above prior art publications, many attempts have been conducted to develop rapidly dissolving formulations, however, none of the attempts have succeeded in developing final dosage formulations that leave no feeling of foreign matter and no residual feeling, achieving enhanced administration convenience.

### [Disclosure]

### [Technical Problem]

It is thus an object of the present invention to provide a formulation for oral administration that is rapidly dissolved in the oral cavity, can be taken without water and is administered in the form of a granule as a final dosage form. Specifically, it is an object of the present invention to provide a novel rapidly dissolving granule formulation that is rapidly dissolved in the oral cavity within 20 seconds after oral administration, leaves no feeling of foreign matter and no residual feeling, and is imparted with a feeling of refreshment. Furthermore, it is an object of the present invention to provide a novel rapidly dissolving granule formulation that is produced by mixing active ingredients with a sugar meeting particular requirements and granulating the mixture wherein the dissolution and absorption of the active ingredient are promoted, and no feeling of foreign matter and no residual feeling are left in the oral cavity.

### [Technical Solution]

To achieve the above object of the present invention, technical solutions are provided as follows.

There is provided a rapidly dissolving granule formulation that is produced by granulation of an active ingredient and at least one rapidly dissolving carrier selected from the group consisting of sugars and sugar alcohols, and is rapidly dissolved in the oral cavity after oral administration. The granule formulation is dissolved in the oral cavity within 20 seconds after administration. The sugars and the sugar alcohols are xylitol, mannitol, isomalt, sorbitol, maltitol, refined sugar, lactose, inositol, erythritol, crystalline fructose, trehalose, ribitol, arabitol, galactitol, lactitol, maltotritol, and mixtures thereof.

The granule formulation includes the rapidly dissolving carrier and the active ingredient in the amount of 40wt%∼99.995wt% and 0.005wt%∼60wt% based on the total formulation, respectively.

The granule formulation is a final dosage form that leaves no feeling of foreign matter and no residual feeling even when taken without water.

A medical product comprising the granule formulation contained in single dosage package unit is provided.

Further, there is provided a rapidly dissolving granule formulation that is produced by granulation of an active ingredient and at least one rapidly dissolving carrier which is rapidly dissolved in the oral cavity.The granule formulation is dissolved in the oral cavity within 20 seconds after administration.

The granule formulation leaves no feeling of foreign matter and no residual feeling even when taken without water.

The rapidly dissolving carrier meets the following requirements:
(1) an instantaneous solubility of at least 30 mg/ml;
(2) a solubility after 5 minutes of at least 50 mg/ml; and
(3) the instantaneous solubility being 90% or less of a maximum solubility.

Also, the rapidly dissolving carrier is sugar or sugar alcohol. The sugars and the sugar alcohols are xylitol, mannitol, isomaltose, sorbitol, maltitol, refined sugar, lactose, inositol, erythritol, crystalline fructose, trehalose, ribitol, arabitol, galactitol, lactitol, maltotritol, and mixtures thereof.

The sugars and the sugar alcohols have below -4kcal/g of heat of dissolution.

The sugars and the sugar alcohols are selected from the group consisting of xylitol, mannitol, isomaltose, sorbitol, maltitol, refined sugar, lactose, inositol, erythritol, crystalline fructose, trehalose, ribitol, arabitol, galactitol, lactitol, maltotritol, and mixtures thereof.

The sugar alcohol can be xylitol.

The granule formulation leaves no feeling of foreign matter and no residual feeling even when taken without water.

A medical product comprising the granule formulation contained in single dosage package unit is provided.

Also, a preparation method of the granule formulation according to the above is provided, the method comprising;
(1) mixing an active ingredient with the rapidly dissolving carrier selected from sugar and sugar alcohols;
(2) providing a binding solution by dissolving pharmaceutically acceptable binder in the solvent;
(3) forming a granule by spraying the solution of (2) to the mixture of (1);
(4) obtaining the granule formulation by mixing the resultant product of (3) with pharmaceutically acceptable excipients.

Otherwise, the method comprising;
(1) preparing a binding solution by dissolving or dispersing an active ingredient and pharmaceutically acceptable binder in the solvent;
(2) forming granule formulation by spraying the binding solution of (1) to the rapidly dissolving carriers selected from sugar and sugar alcohols;
(3) obtaining the final granule formulation by mixing the resultant product of (2) with pharmaceutically acceptable excipients.

The rapidly dissolving carrier used in the above method meets the following requirements:
(1) an instantaneous solubility of at least 30 mg/ml;
(2) a solubility after 5 minutes of at least 50 mg/ml; and
(3) the instantaneous solubility being 90% or less of a maximum solubility.

Also, the rapidly dissolving carrier is sugar or sugar alcohol. The sugars and the sugar alcohols are xylitol, mannitol, isomaltose, sorbitol, maltitol, refined sugar, lactose, inositol, erythritol, crystalline fructose, trehalose, ribitol, arabitol, galactitol, lactitol, maltotritol, and mixtures thereof.

The sugars and the sugar alcohols have below -4kcal/g of heat of dissolution.

The sugar alcohol can be xylitol.

Finally, the preparation method of the granule which can be used in the production of medical product is provided, the method comprising the use of the sugar or sugar alcohols meeting the below requirements.
(1) an instantaneous solubility of at least 30 mg/ml;
(2) a solubility after 5 minutes of at least 50 mg/ml; and
(3) the instantaneous solubility being 90% or less of a maximum solubility.

### [Advantageous Effects]

Unlike conventional granules for oral administration, the novel rapidly dissolving granule formulation of the present invention is rapidly dissolved in the oral cavity after oral administration, leaves no feeling of foreign matter and no residual feeling, and gives a feeling of refreshment, contributing to a marked improvement in administration convenience. Furthermore, the rapidly dissolving granule formulation of the present invention is produced in a relatively simple manner, thus being very advantageous in terms of cost-effectiveness.

### [Best Mode]

The term "granules" as used herein is used as a generic name for powder formulations, fine granule formulations and granule formulations prescribed in the Korean Pharmacopoeia, and it refers to particulate formulations composed of fine particles or general particles. Granules can be produced by two processes, i.e. dry and wet processes. According to the dry process, an active ingredient, an excipient and a binder are mixed to form a slab- or sheet-like material, followed by granulation. According to the wet process, a binder solution is added to an active ingredient or a mixture of an active ingredient and an excipient, and the resulting mixture is mixed using a kneader, followed by granulation and drying. Fluidized bed granulation in which a raw material powder is formed into a fluidized bed using an air current, a binder solution is sprayed onto the fluidized bed, followed by granulation, is mainly used as a wet process. This method is advantageous in that mixing, granulation and drying processes are carried out in a single machine. There is no particular restriction on the method for producing the granule formulation of the present invention, but the use of a fluidized layer granulator is preferred.

The term "feeling of foreign matter" as used herein refers to an unpleasant feeling perceived by a patient who recognizes an orally administered drug as a foreign substance different from substances that the patient usually eats. The feeling of foreign matter is intended to include, for example, a prickly feeling of sand, a feeling of irritation in the oral mucosa and tongue, and a sticky feeling of viscous matter.

The term "residual feeling" as used herein refers to a feeling perceived by a patient who senses as if a formulation including an active ingredient stays partially or entirely in the oral cavity or a trace (e.g., a taste or a feeling) of the formulation remains unremoved although the formulation is already dissolved and absorbed over a long time (e.g. 20 seconds) after oral administration. The residual feeling does not reach a feeling of foreign matter but it also causes an unpleasant feeling for some patients. In the case where a patient feels a residual feeling, the patient may desire to drink water or a beverage. This case is contradictory to the intended purpose of taking a formulation without water. Therefore, a residual feeling, together with a feeling of foreign matter, is a factor that should be taken into consideration in order to improve the quality of a formulation.

The term "feeling of refreshment" as used herein refers to a cool and refreshed feeling perceived by a patient over the entire area of the oral cavity including a topical site where a formulation is located after the formulation has been administered orally. The feeling of refreshment is attributed to an endothermic reaction occurring when a formulation is dissolved in the saliva and is caused by a correlation among heat of dissolution, dissociation energy and dissolution rate of constituent ingredients of the formulation.

The term "instantaneous solubility" as used herein is a new concept defined in the present invention to determine the dissolution pattern of the formulation of the present invention immediately after oral formulation. The instantaneous solubility is calculated as follows. First, 20.0 g of the rapidly dissolving carrier is added to 250 ml of purified water at a temperature of 37 ± 0.5 °C, and then the solubility values of the rapidly dissolving carrier (the weights (mg) of the rapidly dissolving carrier per ml of the purified water) after 1, 3, 5, 10 and 15 minutes are measured at a fixed paddle speed of 50 rpm. The solubility values are plotted. Thereafter, a linear regression curve is fitted according to the least square method and is extrapolated to obtain a value of the Y axis intercept. That is, the instantaneous solubility is a theoretical solubility value when time approaches zero infinite. The concept "instantaneous solubility" was conceived by the present inventors, based on the finding that the formulation of the present invention is rapidly dissolved immediately or within a few seconds after oral administration, and the dissolution effect, the feeling of foreign matter and the residual feeling of the formulation vary depending on the dissolution behaviors of the carrier, including change in the dissolution rate of the carrier at a microscopic level.

The term "solubility after 5 minutes" as used herein refers to a solubility of the rapidly dissolving carrier measured at 5 minutes after 20.0 g of the rapidly dissolving carrier is added to 250 ml of purified water at a temperature of 37 ± 0.5 °C and stirred at a fixed paddle speed of 50 rpm. The concept "solubility after 5 minutes" was conceived by the present inventors, based on the finding that the formulation of the present invention is rapidly dissolved immediately after oral administration, and the dissolution effect, the feeling of foreign matter and the residual feeling of the formulation vary depending on the dissolution behaviors of the carrier, including change in the dissolution rate of the carrier at a microscopic level. The solubility after 5 minutes, together with the instantaneous solubility, is a parameter defined to understand the time-dependent dissolution behaviors of the rapidly dissolving carrier in the oral cavity at a microscopic level.

The term "maximum solubility" as used herein refers to the highest solubility of the rapidly dissolving carrier measured after 20.0 g of the rapidly dissolving carrier is added to 250 ml of purified water at a temperature of 37 ± 0.5 °C and stirred at a fixed paddle speed of 50 rpm. That is, the maximum solubility is 80 mg/ml. The reason why the maximum solubility is defined in the present invention is as follows. Since the rapidly dissolving carrier used in the formulation of the present invention is dissolved immediately after oral administration, it was expected that the higher instantaneous solubility would be more preferable. However, the formulation was unexpectedly found to be poor in terms of a feeling of foreign matter and a residual feeling even at a high instantaneous solubility. Based on this finding, the present inventors established a correlation between the maximum solubility and the instantaneous solubility to set the requirements of the rapidly dissolving carrier that is rapidly dissolved in the oral cavity and leaves no feeling of foreign matter and no residual feeling.

The term "active ingredients" as used herein refers to any pharmacological active ingredients which can be contained in the present granule formulation. Examples of these ingredient include sildenafil, tadalafil, pranlukast hydrate, pseudoephedrine hydrochloride, ranitidine hydrochloride, levocetirizine hydrochloride and pharmaceutically acceptable salts thereof, but not limited thereto.

The term "rapidly dissolving carrier" as used herein refers to a constituent carrier of the formulation of the present invention that is rapidly dissolved after oral administration, leaves no feeling of foreign matter and no residual feeling, and can give a feeling of refreshment. It is particularly preferred that the instantaneous solubility, the solubility after 5 minutes and the heat of dissolution of the rapidly dissolving carrier are at least 30 mg/ml, at least 50 mg/ml and -4 Kcal/g or less, respectively. The instantaneous solubility of the rapidly dissolving carrier is preferably 90% or less of the maximum solubility thereof. The rapidly dissolving carrier meeting all of the requirements is suitable for accomplishing the desired effects of the present invention. The rapidly dissolving carrier is not especially limited so long as it is pharmaceutically acceptable, is rapidly dissolved in the oral cavity, leaves no feeling of foreign matter and no residual feeling, and can give a feeling of refreshment. The rapidly dissolving carrier is preferably a sugar or a sugar alcohol. Examples of such rapidly dissolving carriers include, but are not limited to, pharmaceutically acceptable sugars and sugar alcohols, such as xylitol, mannitol, isomaltose, sorbitol, maltitol, refined sugar, lactose, inositol, erythritol, crystalline fructose, trehalose, ribitol, arabitol, galactitol, lactitol, and maltotritol. These rapidly dissolving carriers may be used alone or as a mixture thereof. Any sugar or sugar alcohol meeting the requirements defined in the present invention may be used without limitation, based on the compatibility with the active ingredient and other pharmaceutical considerations. The present invention will now be explained in detail.

The novel rapidly dissolving granule formulation of the present invention enables administration of the various active ingredients without water, achieves greatly enhanced administration convenience, and does not suffer from the inconvenience of orally rapidly disintegrating tablets and strip formulations capable of being taken without water.The most popular formulations for oral administration are tablets and capsules that are taken along with water. However, according to the medical use, the dosage period or administration situation that are irregularly administered and require a short onset time, preferred are easy-to-carry formulations that can be taken without water. Under such circumstances, research and development has been conducted on orally rapidly disintegrating tablets and strip formulations. Contrary to the expectation that an orally rapidly disintegrating tablet will be rapidly disintegrated in the oral cavity, a patient should hold an orally rapidly disintegrating tablet in the mouth for a considerable time with an unavoidable feeling of foreign matter and the patient feels a residual feeling even after the tablet has been dissolved, which causes the patient to drink a beverage. For this reason, many patients tend to take orally rapidly disintegrating tablets along with water, which is contradictory to intended goals of the present invention. An orally rapidly disintegrating tablet is basically produced by compressing an active ingredient together with a rapidly disintegrating excipient at a low pressure. However, the tablet tends to break during storage or distribution. This necessitates additional packaging of the tablet, which is undesirable in terms of cost-effectiveness. Further, when an ODT comprising a bitter taste active ingredient is located at a certain point of oral cavity, the concentration of the active ingredient becomes high at this point. Therefore, it is actually difficult to make the patient not to feel the bitter taste at all even though the bitter taste is masked.

Another representative formulation capable of being taken without water is a strip formulation produced by incorporating an active ingredient in a film-like strip made of a film-forming polymer. The strip formulation is more rapidly dissolved than an orally rapidly disintegrating tablet but the amount of the active ingredient loaded therein is basically limited. The strip formulation is sensitive to humidity, which increases the possibility of severe distortion or deformation during storage after production. Therefore, special care should be taken in packaging the formulation. Like an orally rapidly disintegrating tablet, the strip formulation encounters a technical difficulty in that additional means is needed to mask the bitterness of a drug. The stickiness of the film-forming polymer constituting the strip formulation leaves a feeling of foreign matter in the oral cavity after administration. If the formulation sticks to the roof of the patient's mouth, which is an unintended location, after administration, the location is difficult to change, thus failing to achieve greatly enhanced administration convenience.

On the other hand, since most conventional granule formulations are dissolved or suspended in beverage or water before administration, they are not substantially classified into formulations that can be taken without water. If a granule formulation needing no mixing with beverage or water for administration is taken without water, a feeling of foreign matter and a residual feeling are severely left in the oral cavity, inevitably leading to deterioration of medication compliance.

Thus, the present inventors have succeeded in developing a novel rapidly dissolving pharmaceutical granule formulation that can overcome the drawbacks of orally rapidly disintegrating tablets, strip formulations and conventional granule formulations. Surprisingly, the present inventors have found that when a rapidly dissolving carrier meeting particular requirements is used, no feeling of foreign matter and no residual feeling are left compared to orally rapidly disintegrating tablets and strip formulations. The present invention has been accomplished based on this finding.

Specifically, the present invention discloses a novel rapidly dissolving granule formulation that is produced by mixing an active ingredient with a rapidly dissolving carrier and wet granulating the mixture with a solution of a pharmaceutically acceptable binder. Alternatively, the rapidly dissolving granule formulation of the present invention may be produced by wet granulating a rapidly dissolving carrier with a solution or dispersion of a pharmaceutically acceptable binder and active ingredient. The rapidly dissolving carrier is preferably a sugar or a sugar alcohol, but is not limited thereto. Particularly preferred is a sugar or a sugar alcohol that meets the following requirements: (1) an instantaneous solubility of at least 30 mg/ml, (2) a solubility after 5 minutes of at least 50 mg/ml, and (3) the instantaneous solubility being 90% or less of a maximum solubility.

The instantaneous solubility of the rapidly dissolving carrier is calculated as follows. First, 20.0 g of the rapidly dissolving carrier is added to 250 ml of purified water at a temperature of 37 ± 0.5 °C, and then the solubility values of the rapidly dissolving carrier after 1, 3, 5, 10 and 15 minutes are measured at a fixed paddle speed of 50 rpm. The solubility values at 1, 3 and 5 minutes are plotted. Thereafter, a linear regression curve is fitted according to the least square method and is extrapolated to obtain a value of the Y axis intercept. That is, the instantaneous solubility is a theoretical solubility value when time approaches zero infinite. The solubility after 5 minutes means the solubility of the rapidly dissolving carrier measured at 5 minutes. The maximum solubility means the highest solubility of the rapidly dissolving carrier under the same conditions as described above.

The present inventors have discovered that when the rapidly dissolving carrier has an instantaneous solubility of at least 30 mg/ml and a solubility after 5 minutes of at least 50 mg/ml, the granule formulation of the present invention is rapidly dissolved in the oral cavity without leaving a feeling of foreign matter and a residual feeling, leading to extreme enhancement of administration convenience. The present inventors have also discovered that when the rapidly dissolving carrier meets the requirements in terms of instantaneous solubility and solubility after 5 minutes but the instantaneous solubility exceeds 90% of the maximum solubility, the granule formulation leaves a feeling of foreign matter and a residual feeling. Particularly, the present inventors have become aware that the two requirements (i.e. rapid dissolution, and no feeling of foreign matter and no residual feeling) of the formulation according to the present invention are associated with not only the solubility of the carrier but also a change in the dissolution rate of the carrier at a microscopic level (that is, despite a high solubility of the carrier, a low dissolution rate or a slow change in dissolution rate at a microscopic level leads to a feeling of foreign matter or a residual feeling). Thus, the present inventors introduced the concept "instantaneous solubility" as a parameter for simultaneously evaluating the dissolution rate and solubility to reflect time-dependent factors at a microscopic level.

Therefore, it should be noted that if the same kind of carrier does not meet the requirements in term of instantaneous solubility and solubility after 5 minutes defined in the present invention, it may be dissolved at a low rate in the oral cavity or may leave a feeling of foreign matter and a residual feeling despite its high dissolution rate in the oral cavity, thus failing to accomplish the desired effects of the present invention.

Specific examples of rapidly dissolving carriers suitable for use in the present invention include, but are not limited to, pharmaceutically acceptable sugars and sugar alcohols, such as xylitol, mannitol, isomaltose, sorbitol, maltitol, refined sugar, lactose, inositol, erythritol, crystalline fructose, trehalose, ribitol, arabitol, galactitol, lactitol and maltotritol. These rapidly dissolving carriers may be used alone or as a mixture thereof. Any sugar or sugar alcohol whose instantaneous solubility and solubility after 5 minutes are at least 30 mg/ml and at least 50 mg/ml, respectively, and whose instantaneous solubility is 90% or less of a maximum solubility may be used to accomplish the desired effects of the present invention, namely, that there is no feeling of foreign matter and no residual feeling. If the rapidly dissolving carrier has an instantaneous solubility of less than 30 mg/ml or a solubility after 5 minutes of less than 50 mg/ml or the instantaneous solubility of the rapidly dissolving carrier exceeds 90% of a maximum solubility, the rapidly dissolving carrier may not be rapidly dissolved in the oral cavity or may leave a feeling of foreign matter and/or a residual feeling in the oral cavity. On the other hand, the dissolution rate of the rapidly dissolving carrier may vary depending on particle size, particle size distribution or specific surface area thereof. However, it is to be noted that the rapidly dissolving carrier can be used without particular limitation of the specific surface area and particle size thereof so long as it meets the requirements in terms of instantaneous solubility and solubility after 5 minutes. For example, the sugar alcohol may be subjected to special spray drying to impart porosity. In this case, the porous sugar alcohol may have a high instantaneous solubility exceeding 90% of a maximum solubility due to its small particle size, leaving a feeling of foreign matter and a residual feeling. Therefore, the porous sugar alcohol is not suitable for use as the rapidly dissolving carrier. The requirements associated with instantaneous solubility and solubility after 5 minutes are concepts different from the specific surface area and particle size of the rapidly dissolving carrier. If the specific surface area is large or the particle size is small but the requirements defined in the present invention are not met, the desired effects of the present invention cannot be accomplished. It should be noted that an increase in dissolution rate resulting from a large specific surface area of the carrier is clearly distinguished from the use of the rapidly dissolving carrier meeting the requirements defined in the present invention.

From the standpoint of imparting a feeling of refreshment, the rapidly dissolving carrier preferably has a heat of dissolution of -4.0 kcal/g or less. For example, xylitol having a heat of dissolution of -36.6 kcal/g is preferred in terms of a feeling of refreshment.

The rapidly dissolving carrier may be present in an amount of 40 to 99.995% by weight, based on the weight of the granule formulation of the present invention.

The active ingredient which can be contained in the present granule formulation is not limited. Examples of the active ingredient can be sildenafil, tadalafil, pranlukast hydrate, pseudoephedrine hydrochloride, ranitidine hydrochloride, levocetirizine hydrochloride and pharmaceutically acceptable salts thereof.

Preferably, active ingredient which needs to be administered urgently in many unexpected situations, such as the ingredients for treatment of erectile dysfunction or migraine can be contained in the present granule formulation. Such ingredients is required to be administered not regularly but urgently when the symptom is appeared even though no water is around. Therefore, the advantage of the present invention can be utilized to the maximum.

The active ingredient can be present in an amount of 0.005 to 60% by weight, based on the weight of the granule formulation of the present invention.

The formulation of the present invention is produced by the following procedure. First, the rapidly dissolving carrier is mixed with active ingredient. The mixture is granulated in a granulator while a solution of a pharmaceutically acceptable binder in a solvent is sprayed thereonto. Subsequently, the granules thus produced are mixed with at least one pharmaceutically acceptable excipient, such as a sweetener or a flavor. The resulting mixture is placed in a packaging container to obtain a granule formulation for one-time administration. Alternatively, the formulation of the present invention may be produced by the following procedure. First, a pharmaceutically acceptable binder and active ingredient are mixed/suspended in a solvent. The rapidly dissolving carrier is granulated in a granulator while the mixture/suspension is sprayed thereonto. Subsequently, the granules thus produced are mixed with at least one pharmaceutically acceptable excipient, such as a sweetener or a flavor. The resulting mixture is put into a packaging container to obtain a granule formulation for one-time administration. One bag of the granule formulation for one-time administration can be administered as a single dose. The granule formulation leaves no feeling of foreign matter and no residual feeling even when taken without water. In addition, the granule formulation gives a feeling of refreshment, eliminating the desire to drink water after administration.

The granule formulation of the present invention can be supplied as easy-to-carry sachets and three-sided packages (stick forms), but is not limited to these packaging types. A patient can carry the packaged granule formulation in a purse and can take it whenever and wherever the patient wants.

In any of the production methods, the binder is commonly used to facilitate the formation of particles during granulation. Examples of such binders include pharmaceutically acceptable ones, such as hydroxylpropyl methylcellulose (HPMC), hydroxypropyl cellulose (HPC), polyvinylpyrrolidone (PVP) and hydroxyethyl cellulose. These binders may be used alone or as a mixture thereof. The binder may be used in the form of a solution in purified water. The binder may be used in an amount of 0.05 to 15% by weight, based to the total weight of the formulation.

In the present invention, one or more pharmaceutically acceptable flavoring agents may be used to improve the flavor of the formulation and to increase the medication compliance of the formulation. Examples of such pharmaceutically acceptable flavoring agents include orange, grape, apple, lemon, strawberry, cherry, pineapple, banana, tutti-frutti, blueberry, peppermint, cocoa, peach and milk flavoring agents. These flavoring agents may be used alone or as a mixture thereof. The flavoring agents may be used in an amount of 0.001 to 10% by weight, based on the total weight of the formulation.

In the present invention, one or more sweeteners may be used to enhance the sweetness of the formulation and to increase the medication compliance of the formulation. The sweeteners are important ingredients in direct connection with the administration convenience of formulations for oral administration. The formulation of the present invention uses a sugar or a sugar alcohol as the rapidly dissolving carrier, which acts as a sweetener. In addition to the sugar or sugar alcohol, natural sweeteners and synthetic sweeteners may also be used. Non-limiting examples of such sweeteners include: pharmaceutically acceptable natural sweeteners, such as sucrose, dextrose, fructose, glucose, liquid glucose and maltose; and pharmaceutically acceptable synthetic sweeteners, such as saccharine, cyclamate, aspartame, acesulfame potassium (K), sucralose, alitame and neotame. The sweeteners may be used in an amount of 0.05 to 20% by weight, based on the total weight of the formulation.

### [Examples]

The present invention will be explained with reference to the following examples. However, these examples are provided for illustrative purposes only and are not intended to limit the scope of the invention. It is to be noted that various modifications can be made in the present invention without departing from the spirit and scope of the invention.

### Preparative Examples 1-33

Large crystals of the sugars and sugar alcohols (xylitol, sorbitol, maltitol, mannitol, isomaltose, inositol, lactose, refined sugar, crystalline fructose, trehalose and erythritol) shown in Table 1 were pulverized into smaller pieces, followed by sieving to prepare rapidly dissolving carriers (Preparative Examples 1-33). The D50 values (expressed in µm) of the rapidly dissolving carriers are shown in Table 1.

**[TABLE 1]**

| Preparative Example No. | Ingredient | D50 |
|---|---|---|
| Preparative Example 1 | Xylitol | 63.4 |
| Preparative Example 2 | Xylitol | 150.6 |
| Preparative Example 3 | Xylitol | 370.6 |
| Preparative Example 4 | Sorbitol | 96.1 |
| Preparative Example 5 | Sorbitol | 254.2 |
| Preparative Example 6 | Sorbitol | 751.6 |
| Preparative Example 7 | Maltitol | 25.4 |
| Preparative Example 8 | Maltitol | 108.1 |
| Preparative Example 9 | Maltitol | 235.2 |
| Preparative Example 10 | Mannitol | 40.0 |
| Preparative Example 11 | Mannitol | 141.5 |
| Preparative Example 12 | Mannitol | 381.1 |
| Preparative Example 13 | Isomaltose | 47.4 |
| Preparative Example 14 | Isomaltose | 222.3 |
| Preparative Example 15 | Isomaltose | 390.2 |
| Preparative Example 16 | Inositol | 49.2 |
| Preparative Example 17 | Inositol | 86.8 |
| Preparative Example 18 | Inositol | 273.7 |
| Preparative Example 19 | Lactose | 30.7 |
| Preparative Example 20 | Lactose | 112.0 |
| Preparative Example 21 | Lactose | 192.2 |
| Preparative Example 22 | Refined sugar | 56.9 |
| Preparative Example 23 | Refined sugar | 319.7 |
| Preparative Example 24 | Refined sugar | 413.9 |
| Preparative Example 25 | Crystalline fructose | 144.0 |
| Preparative Example 26 | Crystalline fructose | 312.9 |
| Preparative Example 27 | Crystalline fructose | 468.9 |
| Preparative Example 28 | Trehalose | 129.7 |
| Preparative Example 29 | Trehalose | 326.8 |
| Preparative Example 30 | Trehalose | 512.0 |
| Preparative Example 31 | Erythritol | 85.3 |
| Preparative Example 32 | Erythritol | 447.8 |
| Preparative Example 33 | Erythritol | 648.3 |

### Test Example 1

In this example, the instantaneous solubility values of the rapidly dissolving carriers prepared in Preparative Examples 1-33 were measured. First, 20.0 g of each sample was placed on a weighing dish and was slowly added to 250 ml of purified water at 37 ± 0.5°C with stirring at a paddle speed at 50 rpm. The solubility values of the sample after 1, 3, 5, 10 and 15 minutes were measured under the conditions shown in Table 2 and analyzed by the method shown in Table 3. The solubility values at the respective dissolution times are shown in Table 4.

**[TABLE 2]**

| Parameter | Condition |
|---|---|
| Sample weight (g) | 20 |
| Dissolution test method | Dissolution test method II described in the Korean Pharmacopoeia |
| Solution volume (mL | 250 |
| Solution temperature (°C) | 37 ± 0.5 |
| Paddle speed (rpm) | 50 |
| Sampling time (min) | 1,3, 5, 10, 15 |
| Sample volume (mL) | Each 15 (filtered after sampling) |

**[TABLE 3]**

| Parameter | | Condition |
|---|---|---|
| Test solution preparation | | 0.3 mL of each sample was diluted to 25 mL with distilled water, and filtered |
| Standard solution preparation | | 24 mg of each standard was precisely weighed and was diluted to 25 mL with distilled water |
| HPLC conditions | Detector | ELSD (Temp.: 80 °C, Gas: N₂ 25 mL/min) |
| | Mobile phase | 30% acetonitrile |
| | Flow rate | 1 mL/min |
| | Column | Prevail carbohydrate ES. 250 x 4.6 mm |
| | Column temperature | 40 °C |
| | Loading volume | 10 µL |

**[TABLE 4]**

| Preparative Example No, | 1 min | 3 min | 5 min |
|---|---|---|---|
| Preparative Example 1 | 67 | 77 | 77 |
| Preparative Example 2 | 51 | 70 | 74 |
| Preparative Example 3 | 22 | 34 | 44 |
| Preparative Example 4 | 73 | 72 | 76 |
| Preparative Example 5 | 54 | 75 | 77 |
| Preparative Example 6 | 34 | 54 | 66 |
| Preparative Example 7 | 49 | 57 | 61 |
| Preparative Example 8 | 47 | 61 | 72 |
| Preparative Example 9 | 28 | 41 | 50 |
| Preparative Example 10 | 64 | 71 | 73 |
| Preparative Example 11 | 74 | 77 | 76 |
| Preparative Example 12 | 33 | 45 | 49 |
| Preparative Example 13 | 58 | 66 | 71 |
| Preparative Example 14 | 56 | 74 | 79 |
| Preparative Example 15 | 36 | 46 | 48 |
| Preparative Example 16 | 58 | 74 | 80 |
| Preparative Example 17 | 40 | 54 | 73 |
| Preparative Example 18 | 23 | 47 | 52 |
| Preparative Example 19 | 40 | 74 | 76 |
| Preparative Example 20 | 36 | 42 | 51 |
| Preparative Example 21 | 29 | 37 | 45 |
| Preparative Example 22 | 44 | 52 | 60 |
| Preparative Example 23 | 24 | 36 | 46 |
| Preparative Example 24 | 23 | 35 | 45 |
| Preparative Example 25 | 47 | 61 | 71 |
| Preparative Example 26 | 35 | 44 | 52 |
| Preparative Example 27 | 21 | 32 | 40 |
| Preparative Example 28 | 57 | 66 | 72 |
| Preparative Example 29 | 37 | 49 | 58 |
| Preparative Example 30 | 15 | 29 | 39 |
| Preparative Example 31 | 44 | 53 | 58 |
| Preparative Example 32 | 13 | 34 | 51 |
| Preparative Example 33 | 10 | 22 | 41 |

The solubility values at 1, 3 and 5 minutes shown in Table 4 were plotted. Thereafter, linear regression curves were fitted according to the least square method and were extrapolated to obtain values of the Y axis intercept. These values were defined as instantaneous solubility values. The instantaneous solubility values and solubility values after 5 minutes of the rapidly dissolving carriers prepared in Preparative Examples 1-33 are shown in Table 5. A judgment was made as to whether the rapidly dissolving carriers met the requirements: instantaneous solubility ≥ 30 mg/ml, solubility after 5 minutes ≥ 50 mg/ml. The results are shown in Table 5.

**[TABLE 5]**

| Preparative Example No. | Instantaneous solubility | Solubility after 5 minutes | Whether or not the requirements of the present invention were met? |
|---|---|---|---|
| Preparative Example 1 | 66.2 | 77 | ○ |
| Preparative Example 2 | 47.8 | 74 | ○ |
| Preparative Example 3 | 16.8 | 44 | × |
| Preparative Example 4 | 71.4 | 76 | ○ |
| Preparative Example 5 | 51.4 | 77 | ○ |
| Preparative Example 6 | 27.3 | 66 | × |
| Preparative Example 7 | 46,7 | 61 | ○ |
| Preparative Example 8 | 41.3 | 72 | ○ |
| Preparative Example 9 | 23,2 | 50 | × |
| Preparative Example 10 | 62,5 | 73 | ○ |
| Preparative Example 11 | 74,2 | 76 | × (instantaneous solubility > 90% of maximum solubility) |
| Preparative Example 12 | 30,3 | 49 | × |
| Preparative Example 13 | 55.3 | 71 | ○ |
| Preparative Example 14 | 52.4 | 79 | ○ |
| Preparative Example 15 | 34.3 | 48 | × |
| Preparative Example 16 | 54.2 | 80 | ○ |
| Preparative Example 17 | 30.9 | 73 | ○ |
| Preparative Example 18 | 18.9 | 52 | × |
| Preparative Example 19 | 36.3 | 76 | ○ |
| Preparative Example 20 | 31.8 | 51 | ○ |
| Preparative Example 21 | 25,0 | 45 | × |
| Preparative Example 22 | 40.0 | 60 | ○ |
| Preparative Example 23 | 18,8 | 46 | × |
| Preparative Example 24 | 17.8 | 45 | × |
| Preparative Example 25 | 41.7 | 71 | ○ |
| Preparative Example 26 | 30.9 | 52 | ○ |
| Preparative Example 27 | 16.8 | 40 | × |
| Preparative Example 28 | 53.8 | 72 | ○ |
| Preparative Example 29 | 32.3 | 58 | ○ |
| Preparative Example 30 | 9.7 | 39 | × |
| Preparative Example 31 | 41.2 | 58 | ○ |
| Preparative Example 32 | 4.2 | 51 | × |
| Preparative Example 33 | 1.1 | 41 | × |

### Examples 1

In accordance with the compositions shown in Tables 6, hypromellose and polyvinylpyrrolidone were dissolved in purified water, and then the solution was sprayed onto a mixture of sildenafil free base and each of the rapidly dissolving carriers prepared in Preparative Examples 2 in a fluidized bed granulator (Glatt, Germany) to obtain granules. The granules were mixed with sucralose and peppermint flavor to produce final granules.

**[Table 6]**

| Ingredients | | Content(mg/formulation) |
|---|---|---|
| Main ingredient | Sidenafil free base | 50 |
| Rapidlly dissolving carriers | Prep. Exam 2 | 390.3 |
| Binder | Hypromellose | 5.0 |
| Binder | Polyvinylpyrrolidone | 1.3 |
| Solvent | Purified water | 87.5 |
| Sweetener | Sucralose | 2.3 |
| Flavouring agent | Peppermint flavour | 1.1 |
| Total amount | | 450 |

### Comparative Example 1

In accordance with the composition shown in Table 7, sildenafil free base, corn starch, crospovidone, hydroxypropyl cellulose, Ludiflash, Prosolv and citric acid hydrate were sieved via a 26 mesh screen (600 µm), and mixed. Colloidal silicon dioxide and sodium stearyl fumarate were sieved via a 30 mesh screen (500 µm), and mixed. The two mixtures were mixed. The resulting mixture was compressed using a single tablet press (XENA-I, RAON, KOREA).

**[Table 7]**

| Ingredients | | Content(mg/formulation) |
|---|---|---|
| Main ingredient | Sildenafil free base | 50 |
| Carriers | Corn starch | 31.5 |
| disintegrant | Crospovidone | 40.0 |
| Binder | Hydroxypropylcellulose | 2.3 |
| Excipient | Ludiflash | 73.7 |
| Fluidizing agent | Prosolv | 25.5 |
| Sweetener | Citric acid hydrate | 4.0 |
| Fluidizing agent | Colloidal silicon dioxide | 1.5 |
| Lubricant | Sodium stearyl fumarate | 6.5 |
| Total amount | | 235 |

| | | |
|---|---|---|
| Ludiflash : BASF, GERMANY PROSOLV : JRS, GERMANY | | |

### Comparative Example 2

Granules were produced in the same manner as in Comparative Example 1, except that sildenafil citrate was used as an active ingredient instead of sildenafil free base and the contents of the ingredients were changed as shown in Table 8.

**[Table 8]**

| Ingredients | | Content(mg/formulation) |
|---|---|---|
| Main ingredient | Sildenafil Citrate (corresponding to 50mg of sildenafil free base) | 70.23 |
| Carriers | Corn starch | 31.5 |
| disintegrant | crospovidone | 40.0 |
| Binder | Hydroxypropylcellulose | 2.27 |
| Excipient | Ludiflash | 53.5 |
| Fluidizing agent | Prosolv | 25.5 |
| Sweetener | Citric acid hydrate | 4.0 |
| Fluidizing agent | Colloidal silicon dioxide | 1.5 |
| Lubricant | Sodium stearyl fumarate | 6.5 |
| Total amount | | 235 |

### Examples 2 to 34

In accordance with the compositions shown in Tables 9 to 12, hypromellose and polyvinylpyrrolidone were dissolved in purified water, and then the solution was sprayed onto a mixture of pranlukast hydrate and each of the rapidly dissolving carriers prepared in Preparative Examples 1 to 33 in a fluidized bed granulator (Glatt, Germany) to obtain granules. The granules were mixed with sucralose and peppermint flavor to produce final granules.

**[Table 9]**

| Ingredients | | contents(mg/formulation) | | | | | | | | |
|---|---|---|---|---|---|---|---|---|---|---|
| | | Ex. 2 | Ex. 3 | Ex. 4 | Ex. 5 | Ex. 6 | Ex. 7 | Ex. 8 | Ex. 9 | Ex. 10 |
| Active ingredient | Pranlukast Hydrate | 50.0 | 50.0 | 50.0 | 50.0 | 50.0 | 50.0 | 50.0 | 50.0 | 50.0 |
| Rapidly dissolving carrier | Prep. Ex. 1 | 390.3 | - | - | - | - | - | - | - | - |
| | Prep. Ex. 2 | - | 390.3 | - | - | - | - | - | - | - |
| | Prep. Ex. 3 | - | - | 390.3 | - | - | - | - | - | - |
| | Prep. Ex. 4 | - | - | - | 390.3 | - | - | - | - | - |
| | Prep. Ex. 5 | - | - | - | - | 390.3 | - | - | - | - |
| | Prep. Ex. 6 | - | - | - | - | - | 390.3 | - | - | - |
| | Prep. Ex. 7 | - | - | - | - | - | - | 390.3 | - | - |
| | Prep. Ex. 8 | - | - | - | - | - | - | - | 390.3 | - |
| | Prep. Ex. 9 | - | - | - | - | - | - | - | - | 390.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 10]**

| Ingredients | | contents(mg/formulation) | | | | | | | | |
|---|---|---|---|---|---|---|---|---|---|---|
| | | Ex. 11 | Ex. 12 | Ex. 13 | Ex. 14 | Ex. 15 | Ex. 16 | Ex. 17 | Ex. 18 | Ex. 19 |
| Active ingredient | Pranlukast Hydrate | 50.0 | 50.0 | 50.0 | 50.0 | 50.0 | 50.0 | 50.0 | 50.0 | 50.0 |
| Rapidly dissolving carrier | Prep. Ex. 10 | 390.3 | - | - | - | - | - | - | - | - |
| | Prep. Ex. 11 | - | 390.3 | - | - | - | - | - | - | - |
| | Prep. Ex. 12 | - | - | 390.3 | - | - | - | - | - | - |
| | Prep. Ex. 13 | - | - | - | 390.3 | - | - | - | - | - |
| | Prep. Ex. 14 | - | - | - | - | 390.3 | - | - | - | - |
| | Prep. Ex. 15 | - | - | - | - | - | 390.3 | - | - | - |
| | Prep. Ex. 16 | - | - | - | - | - | - | 390.3 | - | - |
| | Prep. Ex. 17 | - | - | - | - | - | - | - | 390.3 | - |
| | Prep. Ex. 18 | - | - | - | - | - | - | - | - | 390.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 11]**

| Ingredients | | contents(mg/formulation) | | | | | | | | |
|---|---|---|---|---|---|---|---|---|---|---|
| | | Ex. 20 | Ex. 21 | Ex. 22 | Ex. 23 | Ex. 24 | Ex. 25 | Ex. 26 | Ex. 27 | Ex. 28 |
| Active ingredient | Pranlukast Hydrate | 50.0 | 50.0 | 50.0 | 50.0 | 50.0 | 50.0 | 50.0 | 50.0 | 50.0 |
| Rapidly dissolving carrier | Prep. Ex. 19 | 390.3 | - | - | - | - | - | - | - | - |
| | Prep. Ex. 20 | - | 390.3 | - | - | - | - | - | - | - |
| | Prep. Ex. 21 | - | - | 390.3 | - | - | - | - | - | - |
| | Prep. Ex. 22 | - | - | - | 390.3 | - | - | - | - | - |
| | Prep. Ex. 23 | - | - | - | - | 390.3 | - | - | - | - |
| | Prep. Ex. 24 | - | - | - | - | - | 390.3 | - | - | - |
| | Prep. Ex. 25 | - | - | - | - | - | - | 390.3 | - | - |
| | Prep. Ex. 26 | - | - | - | - | - | - | - | 390.3 | - |
| | Prep. Ex. 27 | - | - | - | - | - | - | - | - | 390.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 12]**

| Ingredients | | contents(mg/formulation) | | | | | |
|---|---|---|---|---|---|---|---|
| | | Ex. 29 | Ex. 30 | Ex. 31 | Ex. 32 | Ex. 33 | Ex. 34 |
| Active ingredient | Pranlukast Hydrate | 50.0 | 50.0 | 50.0 | 50.0 | 50.0 | 50.0 |
| Rapidly dissolving carrier | Prep. Ex. 28 | 390.3 | - | - | - | - | - |
| | Prep. Ex. 29 | - | 390.3 | - | - | - | - |
| | Prep. Ex. 30 | - | - | 390.3 | - | - | - |
| | Prep. Ex. 31 | - | - | - | 390.3 | - | - |
| | Prep. Ex. 32 | - | - | - | - | 390.3 | - |
| | Prep. Ex. 33 | - | - | - | - | - | 390.3 |
| Binder | Hypromellose | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 |
| solvent | purified water | 87.5 | 87.5 | 87.5 | 87.5 | 87.5 | 87.5 |
| sweetener | sucralose | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 |
| total amount | | 450 | 450 | 450 | 450 | 450 | 450 |

### Examples 35 to 67

In accordance with the compositions shown in Tables 13 to 16, hypromellose and polyvinylpyrrolidone were dissolved in purified water, and then the solution was sprayed onto a mixture of pseudoephedrine hydrochloride and each of the rapidly dissolving carriers prepared in Preparative Examples 1 to 33 in a fluidized bed granulator (Glatt, Germany) to obtain granules. The granules were mixed with sucralose and peppermint flavor to produce final granules.

**[Table 13]**

| Ingredients | | contents(mg/formulation) | | | | | | | | |
|---|---|---|---|---|---|---|---|---|---|---|
| | | Ex. 35 | Ex. 36 | Ex. 37 | Ex. 38 | Ex. 39 | Ex. 40 | Ex. 41 | Ex. 42 | Ex. 43 |
| Active ingredient | Pseudoephedrine Hydrochloride | 60.0 | 60.0 | 60.0 | 60.0 | 60.0 | 60.0 | 60.0 | 60.0 | 60.0 |
| Rapidly dissolving carrier | Prep. Ex. 1 | 380.3 | - | - | - | - | - | - | - | - |
| | Prep. Ex. 2 | - | 380.3 | - | - | - | - | - | - | - |
| | Prep. Ex. 3 | - | - | 380.3 | - | - | - | - | - | - |
| | Prep. Ex. 4 | - | - | - | 380.3 | - | - | - | - | - |
| | Prep. Ex. 5 | - | - | - | - | 380.3 | - | - | - | - |
| | Prep. Ex. 6 | - | - | - | - | - | 380.3 | - | - | - |
| | Prep. Ex. 7 | - | - | - | - | - | - | 380.3 | - | - |
| | Prep. Ex. 8 | - | - | - | - | - | - | - | 380.3 | - |
| | Prep. Ex. 9 | - | - | - | - | - | - | - | - | 380.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 14]**

| Ingredients | | contents(mg/formulation) | | | | | | | | |
|---|---|---|---|---|---|---|---|---|---|---|
| | | Ex. 44 | Ex. 45 | Ex. 46 | Ex. 47 | Ex. 48 | Ex. 49 | Ex. 50 | Ex. 51 | Ex. 52 |
| Active ingredient | Pseudoephedrine Hydrochloride | 60.0 | 60.0 | 60.0 | 60.0 | 60.0 | 60.0 | 60.0 | 60.0 | 60.0 |
| Rapidly dissolving carrier | Prep. Ex. 10 | 380.3 | - | - | - | - | - | - | - | - |
| | Prep. Ex. 11 | - | 380.3 | - | - | - | - | - | - | - |
| | Prep. Ex. 12 | - | - | 380.3 | - | - | - | - | - | - |
| | Prep. Ex. 13 | - | - | - | 380.3 | - | - | - | - | - |
| | Prep. Ex. 14 | - | - | - | - | 380.3 | - | - | - | - |
| | Prep. Ex. 15 | - | - | - | - | - | 380.3 | - | - | - |
| | Prep. Ex. 16 | - | - | - | - | - | - | 380.3 | - | - |
| | Prep. Ex. 17 | - | - | - | - | - | - | - | 380.3 | - |
| | Prep. Ex. 18 | - | - | - | - | - | - | - | - | 380.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 15]**

| Ingredients | | contents(mg/formulation) | | | | | | | | |
|---|---|---|---|---|---|---|---|---|---|---|
| | | Ex. 53 | Ex. 54 | Ex. 55 | Ex. 56 | Ex. 57 | Ex. 58 | Ex. 59 | Ex. 60 | Ex. 61 |
| Active ingredient | Pseudoephedrine Hydrochloride | 60.0 | 60.0 | 60.0 | 60.0 | 60.0 | 60.0 | 60.0 | 60.0 | 60.0 |
| Rapidly dissolving carrier | Prep. Ex. 19 | 380.3 | - | - | - | - | - | - | - | - |
| | Prep. Ex. 20 | - | 380.3 | - | - | - | - | - | - | - |
| | Prep. Ex. 21 | - | - | 380.3 | - | - | - | - | - | - |
| | Prep. Ex. 22 | - | - | - | 380.3 | - | - | - | - | - |
| | Prep. Ex. 23 | - | - | - | - | 380.3 | - | - | - | - |
| | Prep. Ex. 24 | - | - | - | - | - | 380.3 | - | - | - |
| | Prep. Ex. 25 | - | - | - | - | - | - | 380.3 | - | - |
| | Prep. Ex. 26 | - | - | - | - | - | - | - | 380.3 | - |
| | Prep. Ex. 27 | - | - | - | - | - | - | - | - | 380.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 16]**

| Ingredients | | contents(mg/formulation) | | | | | |
|---|---|---|---|---|---|---|---|
| | | Ex. 62 | Ex. 63 | Ex. 64 | Ex. 65 | Ex. 66 | Ex. 67 |
| Active ingredient | Pseudoephedrine Hydrochloride | 60.0 | 60.0 | 60.0 | 60.0 | 60.0 | 60.0 |
| Rapidly dissolving carrier | Prep. Ex. 28 | 380.3 | - | - | - | - | - |
| | Prep. Ex. 29 | - | 380.3 | - | - | - | - |
| | Prep. Ex. 30 | - | - | 380.3 | - | - | - |
| | Prep. Ex. 31 | - | - | - | 380.3 | - | - |
| | Prep. Ex. 32 | - | - | - | - | 380.3 | - |
| | Prep. Ex. 33 | - | - | - | - | - | 380.3 |
| Binder | Hypromellose | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 |
| solvent | purified water | 87.5 | 87.5 | 87.5 | 87.5 | 87.5 | 87.5 |
| sweetener | sucralose | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 |
| total amount | | 450 | 450 | 450 | 450 | 450 | 450 |

### Examples 68 to 100

In accordance with the compositions shown in Tables 17 to 20, hypromellose and polyvinylpyrrolidone were dissolved in purified water, and then the solution was sprayed onto a mixture of tadalafil and each of the rapidly dissolving carriers prepared in Preparative Examples 1 to 33 in a fluidized bed granulator (Glatt, Germany) to obtain granules. The granules were mixed with sucralose and peppermint flavor to produce final granules.

**[Table 17]**

| Ingredients | | contents(mg/formulation) | | | | | | | | |
|---|---|---|---|---|---|---|---|---|---|---|
| | | Ex. 68 | Ex. 69 | Ex. 70 | Ex. 71 | Ex. 72 | Ex. 73 | Ex. 74 | Ex. 75 | Ex. 76 |
| Active ingredient | tadalafil | 10.0 | 10.0 | 10.0 | 10.0 | 10.0 | 10.0 | 10.0 | 10.0 | 10.0 |
| Rapidly dissolving carrier | Prep. Ex. 1 | 430.3 | - | - | - | - | - | - | - | - |
| | Prep. Ex. 2 | - | 430.3 | - | - | - | - | - | - | - |
| | Prep. Ex. 3 | - | - | 430.3 | - | - | - | - | - | - |
| | Prep. Ex. 4 | - | - | - | 430.3 | - | - | - | - | - |
| | Prep. Ex. 5 | - | - | - | - | 430.3 | - | - | - | - |
| | Prep. Ex. 6 | - | - | - | - | - | 430.3 | - | - | - |
| | Prep. Ex. 7 | - | - | - | - | - | - | 430.3 | - | - |
| | Prep. Ex. 8 | - | - | - | - | - | - | - | 430.3 | - |
| | Prep. Ex. 9 | - | - | - | - | - | - | - | - | 430.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 18]**

| Ingredients | | contents(mg/formulation) | | | | | | | | |
|---|---|---|---|---|---|---|---|---|---|---|
| | | Ex. 77 | Ex. 78 | Ex. 79 | Ex. 80 | Ex. 81 | Ex. 82 | Ex. 83 | Ex. 84 | Ex. 85 |
| Active ingredient | tadalafil | 10.0 | 10.0 | 10.0 | 10.0 | 10.0 | 10.0 | 10.0 | 10.0 | 10.0 |
| Rapidly dissolving carrier | Prep. Ex. 10 | 430.3 | - | - | - | - | - | - | - | - |
| | Prep. Ex. 11 | - | 430.3 | - | - | - | - | - | - | - |
| | Prep. Ex. 12 | - | - | 430.3 | - | - | - | - | - | - |
| | Prep. Ex. 13 | - | - | - | 430.3 | - | - | - | - | - |
| | Prep. Ex. 14 | - | - | - | - | 430.3 | - | - | - | - |
| | Prep. Ex. 15 | - | - | - | - | - | 430.3 | - | - | - |
| | Prep. Ex. 16 | - | | - | - | - | - | 430.3 | - | - |
| | Prep. Ex. 17 | - | - | - | - | - | - | - | 430.3 | - |
| | Prep. Ex. 18 | - | - | - | - | - | - | - | - | 430.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 19]**

| Ingredients | | contents(mg/formulation) | | | | | | | | |
|---|---|---|---|---|---|---|---|---|---|---|
| | | Ex. 86 | Ex. 87 | Ex. 88 | Ex. 89 | Ex. 90 | Ex. 91 | Ex. 92 | Ex. 93 | Ex. 94 |
| Active ingredient | tadalafil | 10.0 | 10.0 | 10.0 | 10.0 | 10.0 | 10.0 | 10.0 | 10.0 | 10.0 |
| Rapidly dissolving carrier | Prep. Ex. 19 | 430.3 | - | - | - | - | - | - | - | - |
| | Prep. Ex. 20 | - | 430.3 | - | - | - | - | - | - | - |
| | Prep. Ex. 21 | - | - | 430.3 | - | - | - | - | - | - |
| | Prep. Ex. 22 | - | - | - | 430.3 | - | - | - | - | - |
| | Prep. Ex. 23 | - | - | - | - | 430.3 | - | - | - | - |
| | Prep. Ex. 24 | - | - | - | - | - | 430.3 | - | - | - |
| | Prep. Ex. 25 | - | - | - | - | - | - | 430.3 | - | - |
| | Prep. Ex. 26 | - | - | - | - | - | - | - | 430.3 | - |
| | Prep. Ex. 27 | - | - | - | - | - | - | - | - | 430.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

**[Table 20]**

| Ingredients | | contents(mg/formulation) | | | | | |
|---|---|---|---|---|---|---|---|
| | | Ex. 95 | Ex. 96 | Ex. 97 | Ex. 98 | Ex. 99 | Ex. 100 |
| Active ingredient | tadalafil | 10.0 | 10.0 | 10.0 | 10.0 | 10.0 | 10.0 |
| Rapidly dissolving carrier | Prep. Ex. 28 | 430.3 | - | - | - | - | - |
| | Prep. Ex. 29 | - | 430.3 | - | - | - | - |
| | Prep. Ex. 30 | - | - | 430.3 | - | - | - |
| | Prep. Ex. 31 | - | - | - | 430.3 | - | - |
| | Prep. Ex. 32 | - | - | - | - | 430.3 | - |
| | Prep. Ex. 33 | - | - | - | - | - | 430.3 |
| Binder | Hypromellose | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 |
| solvent | purified water | 87.5 | 87.5 | 87.5 | 87.5 | 87.5 | 87.5 |
| sweetener | sucralose | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 |
| total amount | | 450 | 450 | 450 | 450 | 450 | 450 |

### Examples 101 to 108

In accordance with the compositions shown in Tables 21, hypromellose and polyvinylpyrrolidone were dissolved in purified water, and then the solution was sprayed onto a mixture of ranitidine hydrochloride and each of the rapidly dissolving carriers prepared in Preparative Examples 1, 9, 12, 13, 15, 22, 25, 27in a fluidized bed granulator (Glatt, Germany) to obtain granules. The granules were mixed with sucralose and peppermint flavor to produce final granules.

**[Table 21]**

| Ingredients | | contents(mg/formulation) | | | | | | | |
|---|---|---|---|---|---|---|---|---|---|
| | | Ex. 101 | Ex. 102 | Ex. 103 | Ex. 104 | Ex. 105 | Ex. 106 | Ex. 107 | Ex. 108 |
| Active ingredient | ranitidine hydrochloride | 84.0 | 84.0 | 84.0 | 84.0 | 84.0 | 84.0 | 84.0 | 84.0 |
| Rapidly dissolving carrier | Prep. Ex. 1 | 356.3 | - | - | - | - | - | - | - |
| | Prep. Ex. 9 | - | 356.3 | - | - | - | - | - | - |
| | Prep. Ex. 12 | - | - | 356.3 | - | - | - | - | - |
| | Prep. Ex. 13 | - | - | - | 356.3 | - | - | - | - |
| | Prep. Ex. 15 | - | - | - | - | 356.3 | - | - | - |
| | Prep. Ex. 22 | - | - | - | - | - | 356.3 | - | - |
| | Prep. Ex. 25 | - | - | - | - | - | - | 356.3 | - |
| | Prep. Ex. 27 | - | - | - | - | - | - | - | 356.3 |
| Binder | Hypromellose | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 |
| total amount | | 450 | 450 | 450 | 450 | 450 | 450 | 450 | 450 |

### Examples 109 to 117

In accordance with the compositions shown in Tables 22, hypromellose and polyvinylpyrrolidone were dissolved in purified water, and then the solution was sprayed onto a mixture of levocetirizine hydrochloride and each of the rapidly dissolving carriers prepared in Preparative Examples 4, 7, 10, 18, 19, 23, 28, 31, 32 in a fluidized bed granulator (Glatt, Germany) to obtain granules. The granules were mixed with sucralose and peppermint flavor to produce final granules.

**[Table 22]**

| Ingredients | | contents(mg/formulation) | | | | | | | | |
|---|---|---|---|---|---|---|---|---|---|---|
| | | Ex. 109 | Ex. 110 | Ex. 111 | Ex. 112 | Ex. 113 | Ex. 114 | Ex. 115 | Ex. 116 | Ex. 117 |
| Active ingredient | levocetirizine hydrochloride | 5.0 | 5.0 | 5.0 | 5.0 | 5.0 | 5.0 | 5.0 | 5.0 | 5.0 |
| Rapidly dissolving carrier | Prep. Ex. 4 | 435.3 | - | - | - | - | - | - | - | - |
| | Prep. Ex. 7 | - | 435.3 | - | - | - | - | - | - | - |
| | Prep. Ex. 10 | - | - | 435.3 | - | - | - | - | - | - |
| | Prep. Ex. 18 | - | - | - | 435.3 | - | - | - | - | - |
| | Prep. Ex. 19 | - | - | - | - | 435.3 | - | - | - | - |
| | Prep. Ex. 23 | - | - | - | - | - | 435.3 | - | - | - |
| | Prep. Ex. 28 | - | - | - | - | - | - | 435.3 | - | - |
| | Prep. Ex. 31 | - | - | - | - | - | - | - | 435.3 | - |
| | Prep. Ex. 32 | - | - | - | - | - | - | - | - | 435.3 |
| Binder | Hypromellose | 5.0 | 5.0 | 5.0 | 5.0 | 5.0 | 5.0 | 5.0 | 5.0 | 5.0 |
| Binder | Polyvinylpyrrolidone | 1.3 | 1.3 | 1.3 | 1.3 | 1.3 | 1.3 | 1.3 | 1.3 | 1.3 |
| solvent | purified water | 87.5 | 87.5 | 87.5 | 87.5 | 87.5 | 87.5 | 87.5 | 87.5 | 87.5 |
| sweetener | sucralose | 2.3 | 2.3 | 2.3 | 2.3 | 2.3 | 2.3 | 2.3 | 2.3 | 2.3 |
| Flavouring agent | Peppermint flavour | 1.1 | 1.1 | 1.1 | 1.1 | 1.1 | 1.1 | 1.1 | 1.1 | 1.1 |
| total amount | | 450 | 450 | 450 | 450 | 450 | 450 | 450 | 450 | 450 |

### Test Example 2

The formulations produced in Examples 1 and Comparative Examples 1, 2 were organoleptically evaluated for dissolution rate, feeling of foreign matter and residual feeling in the oral cavities of six healthy adults, based on the criteria shown in Table 23. The results are shown in Tables 24-25.

**[Table 23]**

| Score | Dissolution Rate | Feeling of foreign matter | Bitter taste | Residual feeling | |
|---|---|---|---|---|---|
| 0 | After 1 g of each of the formulations was administered to the oral cavity, the time required for complete dissolution of the formulation was measured using stopwatch | No feeling of foreign matter | No bitter taste | A | No residual feeling |
| 1 | | Almost no feeling of foreign matter | Almost no bitter taste | | |
| 2 | | Slight feeling of foreign matter | Slight bitter taste | B | Residual feeling |
| 3 | | Feeling of foreign matter | Bitter taste | | |
| 4 | | Feeling of foreign matter and feeling of repulsion | Bitter taste and feeling of repulsion | C | Sever residual feeling |
| 5 | | Severe feeling of foreign matter and feeling of repulsion | Severe bitter taste and feeling of repulsion | | |

**[Table 24]**

| | Ex. 1 | Comparative Ex. 1 | Comparative Ex. 2 |
|---|---|---|---|
| Dissolution time (sec.) | 10.7 | 47.3 | 43.7 |
| Feeling of foreign matter | 0.2 | 1.8 | 1.8 |
| bitter taste | 0.3 | 2.3 | 4.8 |
| Residual Feeling | A | B | C |

**[Table 25]**

| | subject 1 | | | subject 2 | | | subject 3 | | | subject 4 | | | subject 5 | | | subject 6 | | |
|---|---|---|---|---|---|---|---|---|---|---|---|---|---|---|---|---|---|---|
| | Ex .1 | Comparative Ex.11 | Comparative Ex.2 | Ex.1 | Comparative Ex.11 | Comparative Ex.2 | Ex.1 | Comparative Ex.11 | Comparative Ex.2 | Ex.1 | Comparative Ex.1 | Comparative Ex.2 | Ex.1 | Comparative Ex.1 | Comparative Ex.2 | Ex.1 | Comparative Ex.1 | Comparative Ex.2 |
| Dissolution time (sec.) | 11 | 35 | 40 | 12 | 63 | 49 | 12 | 40 | 40 | 10 | 35 | 44 | 9 | 70 | 55 | 10 | 41 | 34 |
| Feeling of foreign matter | 0 | 2 | 2 | 0 | 1 | 1 | 1 | 2 | 2 | 0 | 1 | 1 | 0 | 2 | 2 | 0 | 3 | 3 |
| bitter taste | 0 | 3 | 5 | 0 | 1 | 5 | 1 | 2 | 4 | 0 | 3 | 5 | 1 | 2 | 5 | 0 | 3 | 5 |
| Residual Feeling | A | B | C | A | C | C | A | B | C | A | C | C | A | C | C | A | B | C |

### Test Example 3

Preparative examples 1 to 33 were organoleptically evaluated for dissolution rate, feeling of foreign matter and residual feeling in the oral cavities of six healthy adults, based on the criteria shown in Table 26. The results are shown in Tables 27-29.

**[Table 26]**

| [TABL 14] | | | | |
|---|---|---|---|---|
| Score | Dissolution rate | Feeling of foreign matter | Residual feeling | |
| 0 | After 1 g of each of the formulations was administered to the oral cavity, the time required for complete dissolution of the formulation was measured using stopwatch | No feeling of foreign matter | A | No residual feeling |
| 1 | | Almost no feeling of foreign matter | | |
| 2 | | Slight feeling of foreign matter | B | Residual feeling |
| 3 | | Feeling of foreign matter | | |
| 4 | | Feeling of foreign matter and feeling of repulsion | C | Severe residual feeling |
| 5 | | Severe feeling of foreign matter and feeling of repulsion | | |

**[Table 27]**

| | Prep. Ex. 1 | Prep. Ex. 2 | Prep. Ex. 3 | Prep. Ex. 4 | Prep. Ex. 5 | Prep. Ex. 6 | Prep. Ex. 7 | Prep. Ex. 8 | Prep. Ex. 9 | Prep. Ex. 10 | Prep. Ex. 11 |
|---|---|---|---|---|---|---|---|---|---|---|---|
| dissolution rate (sec.) | 4.8 | 7.2 | 14.7 | 8.0 | 8.0 | 17.3 | 13.0 | 16.3 | 22.8 | 9.0 | 15.0 |
| Feeling of foreign matter | 0.2 | 1.2 | 3.5 | 0.8 | 1.8 | 4.5 | 1.2 | 2.0 | 4.2 | 0.3 | 4.0 |
| Residual feeling | A | A | B | A | A | B | A | A | B | A | B |

**[Table 28]**

| | Prep. Ex. 12 | Prep. Ex. 13 | Prep. Ex. 14 | Prep. Ex. 15 | Prep. Ex. 16 | Prep. Ex. 17 | Prep. Ex. 18 | Prep. Ex. 19 | Prep. Ex. 20 | Prep. Ex. 21 | Prep. Ex. 22 |
|---|---|---|---|---|---|---|---|---|---|---|---|
| dissolution rate (sec.) | 19.7 | 5.7 | 11.2 | 12.3 | 8.5 | 14.7 | 26.0 | 9.0 | 12.0 | 14.0 | 5.8 |
| Feeling of foreign matter | 2.5 | 0.3 | 1.8 | 2.6 | 1.0 | 1.7 | 4.3 | 0.3 | 0.7 | 3.2 | 0.2 |
| Residual feealing | C | A | A | C | A | A | B | A | A | B | A |

**[Table 29]**

| | Prep. Ex. 23 | Prep. Ex. 24 | Prep. Ex. 25 | Prep. Ex. 26 | Prep. Ex. 27 | Prep. Ex. 28 | Prep. Ex. 29 | Prep. Ex. 30 | Prep. Ex. 31 | Prep. Ex. 32 | Prep. Ex. 33 |
|---|---|---|---|---|---|---|---|---|---|---|---|
| dissolution rate (sec.) | 9.3 | 13.2 | 4.7 | 7.5 | 9.8 | 6.3 | 12.3 | 14.7 | 6.7 | 11.2 | 17.7 |
| Feeling of foreign matter | 3.3 | 3.7 | 0.0 | 1.2 | 3.2 | 0.7 | 1.8 | 3.2 | 0.5 | 3.3 | 3.7 |
| Residual feeling | B | B | A | A | B | A | A | B | A | B | B |

### Test Example 4

Examples 2 to 117 were organoleptically evaluated for dissolution rate, feeling of foreign matter and residual feeling in the oral cavities of six healthy adults, based on the criteria shown in Table 30. The results are shown in Table 31.

**[Table 30]**

| [TABL 14] | | | | |
|---|---|---|---|---|
| Score | Dissolution rate | Feeling of foreign matter | Residual feeling | |
| 0 | After 1 g of each of the formulations was administered to the oral cavity, the time required for complete dissolution of the formulation was measured using stopwatch | No feeling of foreign matter | A | No residual feeling |
| 1 | | Almost no feeling of foreign matter | | |
| 2 | | Slight feeling of foreign matter | B | Residual feeling |
| 3 | | Feeling of foreign matter | | |
| 4 | | Feeling of foreign matter and feeling of repulsion | C | Severe residual feeling |
| 5 | | Severe feeling of foreign matter and feeling of repulsion | | |

**[Table 31]**

| Ex. | dissolution rate (sec.) | Feeling of foreign matter | Residual feeling |
|---|---|---|---|
| Ex. 2 | 4.7 | 0.2 | A |
| Ex. 3 | 7.2 | 1.3 | A |
| Ex. 4 | 15.0 | 3.5 | B |
| Ex. 5 | 8.2 | 1.0 | A |
| Ex. 6 | 8.2 | 1.8 | A |
| Ex. 7 | 17.7 | 4.7 | B |
| Ex. 8 | 13.3 | 1.3 | A |
| Ex. 9 | 16.3 | 1.8 | A |
| Ex. 10 | 23.0 | 4.5 | B |
| Ex. 11 | 9.3 | 0.3 | A |
| Ex. 12 | 15.7 | 4.0 | B |
| Ex. 13 | 19.8 | 2.5 | C |
| Ex. 14 | 6.0 | 0.5 | A |
| Ex. 15 | 11.7 | 1.8 | A |
| Ex. 16 | 13.7 | 2.6 | C |
| Ex. 17 | 10.0 | 1.2 | A |
| Ex. 18 | 15.8 | 1.7 | A |
| Ex. 19 | 27.3 | 4.5 | B |
| Ex. 20 | 9.0 | 0.3 | A |
| Ex. 21 | 12.8 | 0.8 | A |
| Ex. 22 | 15.5 | 3.2 | B |
| Ex. 23 | 6.8 | 0.2 | A |
| Ex. 24 | 9.5 | 3.2 | B |
| Ex. 25 | 14.7 | 4.0 | B |
| Ex. 26 | 6.0 | 0.2 | A |
| Ex. 27 | 8.0 | 1.2 | A |
| Ex. 28 | 11.2 | 3.2 | B |
| Ex. 29 | 7.5 | 1.0 | A |
| Ex. 30 | 12.7 | 2.0 | A |
| Ex. 31 | 15.3 | 3.3 | B |
| Ex. 32 | 6.8 | 0.5 | A |
| Ex. 33 | 11.2 | 3.3 | B |
| Ex. 34 | 18.7 | 3.9 | B |
| Ex. 35 | 5.0 | 0.7 | A |
| Ex. 36 | 7.7 | 1.5 | A |
| Ex. 37 | 14.8 | 3.8 | B |
| Ex. 38 | 8.2 | 1.0 | A |
| Ex. 39 | 8.5 | 1.8 | A |
| Ex. 40 | 17.5 | 4.8 | B |
| Ex. 41 | 13.5 | 1.3 | A |
| Ex. 42 | 16.8 | 2.0 | A |
| Ex. 43 | 23.2 | 4.2 | B |
| Ex. 44 | 9.5 | 0.3 | A |
| Ex. 45 | 15.3 | 3.9 | B |
| Ex. 46 | 20.0 | 2.6 | C |
| Ex. 47 | 6.2 | 0.7 | A |
| Ex. 48 | 11.7 | 1.8 | A |
| Ex. 49 | 12.5 | 2.7 | C |
| Ex. 50 | 8.8 | 1.0 | A |
| Ex. 51 | 15.2 | 1.8 | A |
| Ex. 52 | 26.2 | 4.5 | B |
| Ex. 53 | 9.3 | 0.5 | A |
| Ex. 54 | 12.5 | 0.8 | A |
| Ex. 55 | 14.5 | 3.3 | B |
| Ex. 56 | 6.3 | 0.5 | A |
| Ex. 57 | 9.7 | 3.2 | B |
| Ex. 58 | 13.3 | 3.8 | B |
| Ex. 59 | 5.2 | 0.0 | A |
| Ex. 60 | 7.7 | 1.3 | A |
| Ex. 61 | 10.0 | 3.2 | B |
| Ex. 62 | 6.5 | 1.0 | A |
| Ex. 63 | 12.8 | 1.8 | A |
| Ex. 64 | 15.0 | 3.3 | B |
| Ex. 65 | 6.8 | 0.5 | A |
| Ex. 66 | 11.7 | 3.5 | B |
| Ex. 67 | 18.2 | 3.8 | B |
| Ex. 68 | 5.0 | 0.3 | A |
| Ex. 69 | 7.7 | 1.2 | A |
| Ex. 70 | 15.0 | 4.0 | B |
| Ex. 71 | 8.3 | 1.0 | A |
| Ex. 72 | 8.5 | 1.8 | A |
| Ex. 73 | 17.7 | 4.5 | B |
| Ex. 74 | 13.3 | 1.2 | A |
| Ex. 75 | 16.5 | 1.8 | A |
| Ex. 76 | 23.0 | 4.3 | B |
| Ex. 77 | 9.2 | 0.8 | A |
| Ex. 78 | 15.2 | 4.1 | B |
| Ex. 79 | 20.2 | 2.6 | C |
| Ex. 80 | 6.0 | 0.5 | A |
| Ex. 81 | 11.3 | 2.0 | A |
| Ex. 82 | 12.5 | 2.7 | C |
| Ex. 83 | 9.0 | 1.2 | A |
| Ex. 84 | 15.2 | 1.7 | A |
| Ex. 85 | 26.5 | 4.7 | B |
| Ex. 86 | 9.5 | 0.5 | A |
| Ex. 87 | 12.5 | 1.2 | A |
| Ex. 88 | 14.3 | 3.2 | B |
| Ex. 89 | 6.3 | 0.5 | A |
| Ex. 90 | 9.5 | 3.4 | B |
| Ex. 91 | 13.7 | 4.0 | B |
| Ex. 92 | 4.8 | 0.2 | A |
| Ex. 93 | 7.8 | 1.3 | A |
| Ex. 94 | 10.3 | 3.5 | B |
| Ex. 95 | 6.8 | 0.8 | A |
| Ex. 96 | 12.8 | 2.0 | A |
| Ex. 97 | 14.8 | 3.5 | B |
| Ex. 98 | 6.8 | 0.7 | A |
| Ex. 99 | 11.7 | 3.5 | B |
| Ex. 100 | 18.0 | 3.8 | B |
| Ex. 101 | 5.0 | 0.5 | A |
| Ex. 102 | 23.0 | 4.5 | B |
| Ex. 103 | 20.2 | 2.9 | C |
| Ex. 104 | 6.2 | 0.3 | A |
| Ex. 105 | 12.8 | 2.8 | C |
| Ex. 106 | 6.2 | 0.7 | A |
| Ex. 107 | 4.8 | 0.5 | A |
| Ex. 108 | 10.3 | 3.2 | B |
| Ex. 109 | 8.2 | 1.3 | A |
| Ex. 110 | 13.2 | 1.5 | A |
| Ex. 111 | 9.2 | 0.3 | A |
| Ex. 112 | 26.3 | 4.3 | B |
| Ex. 113 | 9.5 | 0.5 | A |
| Ex. 114 | 9.8 | 3.2 | B |
| Ex. 115 | 6.5 | 1.2 | A |
| Ex. 116 | 7.2 | 0.8 | A |
| Ex. 117 | 11.3 | 3.8 | B |

From the above results, it can be seen that the granule formulations produced using the rapidly dissolving carriers failing to meet either of the requirements in terms of instantaneous solubility, solubility after 5 minutes, and the relationship between the instantaneous solubility and the maximum solubility did not achieve the desired effects of the present invention due to their severe feeling of foreign matter and residual feeling.

### [Industrial Applicability]

The novel rapidly dissolving granule formulation of the present invention is rapidly dissolved in the oral cavity, leaves no feeling of foreign matter and no residual feeling, and gives a feeling of refreshment. Particularly, the formulation of the present invention can accomplish the desired effects irrespective of the kind of active ingredient. Therefore, the formulation of the present invention is applicable to various active ingredients. In addition, the formulation of the present invention can be produced in a relatively simple manner. This allows for an economical and efficient production process.

## Claims

1. A rapidly dissolving granule formulation which is obtained by granulation of an active ingredient and at least one rapidly dissolving carrier selected from the group consisting of sugars and sugar alcohols, wherein the granule formulation is rapidly dissolved in the oral cavity after oral administration.

2. The rapidly dissolving granule formulation according to Claim 1, wherein the granule formulation is dissolved within 20 seconds in the oral cavity.

3. The rapidly dissolving granule formulation according to Claim 1 or 2, wherein the sugars or sugar alcohols are selected from the group consisting of xylitol, mannitol, isomalt, sorbitol, maltitol, refined sugar, lactose, inositol, erythritol, crystalline fructose, trehalose, ribitol, arabitol, galactitol, lactitol, maltotritol, and mixtures thereof.

4. The rapidly dissolving granule formulation according to Claim 3, wherein the rapidly dissolving carrier and the active ingredient are in the amount of 40wt%∼99.995wt% and 0.005wt%∼60wt% of the total formulation, respectively.

5. The rapidly dissolving granule formulation according to Claim 4, wherein the formulation is a final dosage form that leaves no feeling of foreign matter and no residual feeling even when taken without water.

6. A medical product comprising the granule formulation according to Claim 5 contained in a single dosage package unit.

7. A rapidly dissolving granule formulation which is obtained by granulation of an active ingredient and at least one rapidly dissolving carrier, wherein the granule formulation is dissolved within 20 seconds in the oral cavity when oral administration.

8. The rapidly dissolving granule formulation according Claim 7, wherein the granule formulation leaves no feeling of foreign matter and no residual feeling even when taken without water.

9. The rapidly dissolving granule formulation according to Claim 8, whereinthe rapidly dissolving carrier meets the following requirements (1), (2) and (3):
(1) an instantaneous solubility of at least 30 mg/ml;
(2) a solubility after 5 minutes of at least 50 mg/ml; and
(3) the instantaneous solubility being 90% or less of a maximum solubility.

10. The rapidly dissolving granule formulation according to Claim 9, wherein the rapidly dissolving carrier is sugar or sugar alcohol.

11. The rapidly dissolving granule formulation according to Claim 10, wherein the sugar or the sugar alcohol has below -4kcal/g of heat of dissolution.

12. The rapidly dissolving granule formulation according to Claim 10, wherein the sugar and the sugar alcohol is selected from the group consisting of xylitol, mannitol, isomalt, sorbitol, maltitol, refined sugar, lactose, inositol, erythritol, crystalline fructose, trehalose, ribitol, arabitol, galactitol, lactitol, maltotritol, and mixtures thereof.

13. The rapidly dissolving granule formulation according to Claim 12, wherein the sugar alcohol is xylitol.

14. The rapidly dissolving granule formulation according to Claim 13, wherein the granule formulation leaves no feeling of foreign matter and no residual feeling, and gives a feeling of refreshment even when taken without water.

15. A medical product comprising the granule formulation according to Claim 9 contained in a single dosage package unit.

16. A preparation method of the granule formulation which is rapidly dissolved in the oral cavity after oral administration, comprising:
(1) mixing an active ingredient with at least one rapidly dissolving carrier selected from sugar and sugar alcohol;
(2) preparing a binding solution by dissolving pharmaceutically acceptable binder in the purified water solvent;
(3) forming granules by spraying the solution of step (2) to the mixture of step (1); and
(4) obtaining the granule formulation by mixing the resultant product of step (3) with at least one pharmaceutically acceptable additive.

17. A preparation method of the granule formulation which is rapidly dissolved in the oral cavity after oral administration, comprising:
(1) preparing a binding solution by dissolving or suspending an active ingredient and at lease a pharmaceutical acceptable binder in a solvent;
(2) forming granules by spraying the solution of step (1) to a rapidly dissolving carrier selected from sugar and sugar alcohol; and
(3) obtaining the granule formulation by mixing the resultant product of step (2) with at least one pharmaceutically acceptable additive.

18. The preparation method of the granule formulation according to Claim 16 or 17, wherein the rapidly dissolving carrier meets the following requirements (1), (2) and (3):
(1) an instantaneous solubility of at least 30 mg/ml;
(2) a solubility after 5 minutes of at least 50 mg/ml; and
(3) the instantaneous solubility being 90% or less of a maximum solubility.

19. The preparation method of the granule formulation according to Claim 18, wherein the rapid dissolving carrier is selected from the group consisting of xylitol, mannitol, isomalt, sorbitol, maltitol, refined sugar, lactose, inositol, erythritol, crystalline fructose, trehalose, ribitol, arabitol, galactitol, lactitol, maltotritol, and mixtures thereof.

20. The preparation method of the granule formulation according to Claim 19, wherein the rapid dissolving carrier has below -4kcal/g of heat of dissolution.

21. The preparation method of the granule formulation according to Claim 20, wherein the rapid dissolving carrier is xylitol.

22. Granules for use for the manufacture of a medicament, wherein the granules are obtained by granulation using sugar or sugar alcohol which meets the following requirements (1), (2) and (3):
(1) an instantaneous solubility of at least 30 mg/ml;
(2) a solubility after 5 minutes of at least 50 mg/ml; and
(3) the instantaneous solubility being 90% or less of a maximum solubility.
